# EUROPÄISCHE PATENTSCHRIFT

(11) **EP 2 867 650 B1**
(45) Veröffentlichungstag und Bekanntmachung des Hinweises auf die Patenterteilung: **24.10.2018**
(21) Anmeldenummer: 13727161.5
(22) Anmeldetag: 06.06.2013
(51) Int. Cl.: G01N 15/06, G01N 33/00, G01M 15/10

(54) **GASSENSOR**
GAS SENSOR
CAPTEUR DE GAZ

(30) Priorität: 27.06.2012 DE 102012211039
(43) Veröffentlichungstag der Anmeldung: 06.05.2015
(73) Patentinhaber: Robert Bosch GmbH, 70442 Stuttgart (DE)
(72) Erfinder: SCHUPPLER, Christian, 70435 Stuttgart-Zuffenhausen (DE); SCHAAF, Martin, 70499 Stuttgart (DE); STORBECK, Karsten, 74081 Heilbronn-Sontheim (DE); SCHROEDER, Thomas, 71277 Rutesheim (DE); RENTSCHLER, Simon, 70199 Stuttgart (DE); LINCK-LESCANNE, Markus, 72827 Wannweil (DE); ECKARDT, Martin, 70197 Stuttgart (DE); HOLZKNECHT, Christopher, 70186 Stuttgart (DE); BRUECK, Marc, 71149 Bondorf (DE)
(86) Internationale Anmeldenummer: PCT/EP2013/061637
(87) Internationale Veröffentlichungsnummer: WO 2014/001049

(56) Entgegenhaltungen:
- WO-A1-2005/017515
- DE-A1-102004 033 958
- DE-A1-102006 035 058
- DE-A1-102008 041 038

## Beschreibung

### Stand der Technik

Die Erfindung geht aus von einem Gassensor zur Bestimmung mindestens einer Zustandsgröße eines Messgases, insbesondere der Partikelkonzentration im Abgas eines Brenners oder einer Brennkraftmaschine, wie er beispielsweise aus der DE-102008041038 A1 bereits bekannt ist.

Die dort gezeigten Abgassensoren weisen an ihrem dem Abgas auszusetzenden Ende ein äußeres und ein inneres Schutzrohr auf, die ein keramisches Sensorelement umgeben. Die Schutzrohre sind derart gestaltet, dass es zu einer Strömungsführung kommt, in der das Sensorelement einerseits vor dem Auftreffen flüssiger Bestandteile des Abgases, zum Beispiel Wassertropfen, geschützt ist und es andererseits zu einer Überströmung eines Messbereiches des Sensorelements kommt, die im Wesentlichen in Längsrichtung des Sensors erfolgt.

Derartige Abgassensoren sind aufgrund des langen Weges des Abgases innerhalb der Schutzrohre und aufgrund der eher geringen Wechselwirkung des Abgases mit dem Messbereiches des Sensorelements bei der Überströmung in Längsrichtung insbesondere zur Montage im Abgastrakten von Brennkraftmaschinen und Brennern vorgesehen, in denen eine relativ hohe Strömungsgeschwindigkeit vorliegt und/oder in Applikationen, in denen die Anforderungen an die Dynamik der Messeinrichtungen eher niedrig sind.

Andererseits besteht aber auch ein Bedarf an Abgassensoren, die auch in Abgastrakten von Brennkraftmaschinen und Brennern, in denen nur eine geringe Strömungsgeschwindigkeit herrscht, mit guter Dynamik messen können. Beispielsweise sollen derartige Sensoren, als Rußsensoren ausgebildet und in einer Abgasbox eines NKWs verbaut, innerhalb einer gewissen Zeit eine Mindestmenge an Ruß aufgesammelt haben, sodass eine präzise Messung der von der Brennkraftmaschine des NKW emittierten Rußmenge möglich ist.

Ein weiterer, ähnlicher Gassensor wird in DE102006035058 beschrieben.

### Vorteile der Erfindung

Die erfindungsgemäßen Gassensoren sind daher so ausgestaltet, dass es zu einer verstärkten Überströmung des Sensorelements kommt. Auch wenn diese Gassensoren Abgasen mit geringer Strömungsgeschwindigkeit ausgesetzt sind, kommt es auf diese Weise zu einer guten Messdynamik.

Die Ausgestaltung des erfindungsgemäßen Gassensors erfolgt nach der in Anspruch 1 definierten Konzeption.

Gemäß der Konzeption aus Anspruch 1 sind erfindungsgemäß ein oder mehrere, zum Beispiel zwei, Gaseintritte vorgesehen, die auf der Mantelfläche des Außenschutzrohrs angeordnet und als Drallklappen ausgebildet sind. Gemäß dieser Konzeption kann es insbesondere vorgesehen sein, dass das Außenschutzrohr an seiner Stirnseite geschlossen ist, also keine Gaseintritte aufweist.

Gemäß einer zweiten Konzeption, die nicht unter den Schutzumfang des Anspruchs fällt, ist vorgesehen, dass das Innenschutzrohr in seiner Mantelfläche einen oder mehrere Gaseintritte aufweist.

Es kann insbesondere vorgesehen sein, dass das Innenschutzrohr nur einen Gaseintritt aufweist und dieser aus einer einzigen Öffnung besteht. Das Einströmen des Gases in den Innenraum des Innenschutzrohrs, also sein Zuströmen zu dem Sensorelement ist dann nur über diesen einen Gaseintritt möglich. Es kann so bewirkt werden, dass das Sensorelement im Inneren des Innenschutzrohrs gerichtet angeströmt wird. In diesem Fall ist bevorzugt, dass der Gassensor als Rußsensor ausgebildet ist und dass der eine Gaseintritt einer Interdigitalelektrode des Sensorelements zugewandt ist.

Abgassensoren gemäß der zweiten Konzeption können insbesondere ein Innenschutzrohr aufweisen, dessen Gaseintritt oder Gaseintritte bezogen auf die Anordnung im Abgastrakt einer Brennkraftmaschine oder eines Brenners lediglich stromabwärts angeordnet sind. Alternativ oder zusätzlich kann vorgesehen sein, dass Abgassensoren gemäß der zweiten Konzeption Außenschutzrohre aufweisen, die ein oder mehrere, zum Beispiel zwei, Gaseintritte aufweisen, und dieser Gaseintritt oder diese Gaseintritte insbesondere als Drallklappen ausgebildet sind, wobei diese Gaseintritte insbesondere bezogen auf die Anordnung im Abgastrakt einer Brennkraftmaschine oder eines Brenners lediglich stromaufwärts angeordnet sind.

Zum gerichteten Verbau des Sensors in einem Abgastrakt können stets entsprechende Mittel vorgesehen sein, die beispielsweise Markierungen, Rastmittel, Überwurfmuttern, Bajonettverschlüsse und/oder ähnliches umfassen.

Gemäß einer dritten Konzeption, die nicht unter den Schutzumfang des Anspruchs fällt, sind Abgassensoren vorgesehen, die lediglich ein Einfach-Schutzrohr aufweisen, also ein Innenschutzrohr, aber kein Außenschutzrohr. Es kann insbesondere vorgesehen sein, dass das Innenschutzrohr nur einen Gaseintritt aufweist und dieser aus einer einzigen Öffnung besteht. Das Einströmen des Gases in den Innenraum des Innenschutzrohrs, also sein Zuströmen zu dem Sensorelement ist dann nur über diesen einen Gaseintritt möglich. Es kann so bewirkt werden, dass das Sensorelement im Inneren des Innenschutzrohrs gerichtet angeströmt wird. In diesem Fall ist bevorzugt, dass der Gassensor als Rußsensor ausgebildet ist und dass der eine Gaseintritt einer Interdigitalelektrode des Sensorelements zugewandt ist.

Abgassensoren gemäß der dritten Konzeption können insbesondere ein Innenschutzrohr aufweisen, dessen Gaseintritt oder Gaseintritte bezogen auf die Anordnung im Abgastrakt einer Brennkraftmaschine oder eines Brenners lediglich stromaufwärts angeordnet sind. Zusätzlich oder alternativ können das Sensorelement und/oder das Gasaustrittsloch bezogen auf eine Mittelachse des Innenschutzrohrs außermittig versetzt angeordnet sein, insbesondere bezogen auf die Anordnung im Abgastrakt einer Brennkraftmaschine oder eines Brenners in stromabwärtige Richtung,
Zum gerichteten Verbau des Sensors in einem Abgastrakt können stets entsprechende Mittel vorgesehen sein, die beispielsweise Markierungen, Rastmittel, Überwurfmuttern, Bajonettverschlüsse und/oder ähnliches umfassen.

Sensoren gemäß der ersten, der zweiten und der dritten Konzeption können zum Beispiel in solchen Vorrichtungen innerhalb eines Abgastrakts eines Brenners oder einer Brennkraftmaschine verbaut sein, in denen der Strömungsquerschnitt erhöht ist und/oder die Strömungsgeschwindigkeit herabgesetzt ist. Erfindungsgemäße Sensoren gemäß der ersten, der zweiten und der dritten Konzeption können beispielsweise in Bypassleitungen eines Abgastrakts. Beispielsweise können Sensoren in einer Abgasbox insbesondere eines NKW verbaut sein.

### Kurze Beschreibung der Zeichnungen

Die Erfindung und weitere beispielhafte Konzeptionen sind anhand von in den Zeichnungen dargestellten Beispielen in der nachfolgenden Beschreibung näher erläutert. Es zeigen:
Fig. 1 zeigt einen Abgassensor gemäß der ersten Konzeption der Erfindung,
Fig. 2 zeigt einen Abgassensor gemäß der beispielhaften zweiten Konzeption,
Fig. 3 zeigt einen Abgassensor gemäß der beispielhaften dritten Konzeption.

### Ausführungsformen

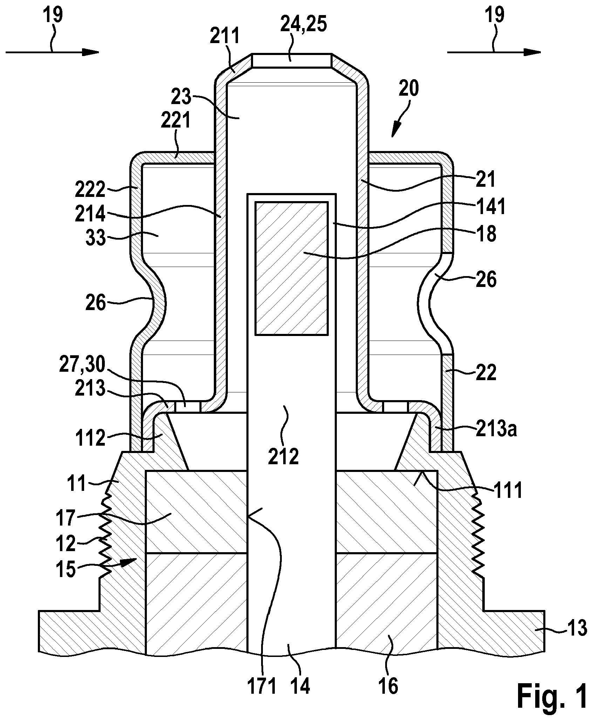

In der Figur 1 ist ein dem Abgas zugewandter Abschnitt eines Gassensors gemäß der ersten Konzeption der Erfindung dargestellt. Es handelt sich um einen Sensor zur Bestimmung der Partikel, insbesondere Rußkonzentration im Abgas einer Brennkraftmaschine, auch Partikel- oder Rußsensor genannt. Er wird als Beispiel für einen allgemeinen Gassensor zur Bestimmung mindestens einer Zustandsgröße eines Messgases angegeben. Andere Gassensoren dieser Art sind Gassensoren zur Bestimmung der Sauerstoffkonzentration im Abgas einer Brennkraftmaschine, sog. Lambdasonden, oder Gassensoren zur Bestimmung der Stickoxidkonzentration im Abgas einer Brennkraftmaschine. Auch Temperaturmessfühler zur Messung der Abgastemperatur können ein solcher Gassensor sein.

Der der Figur 1 dargestellte Gassensor weist ein metallisches Gehäuse 11 auf, das zum Einbau in einen hier nicht dargestellten Strömungskanal für das Messgas, insbesondere in das Abgasrohr eines Brenners oder einer Brennkraftmaschine, mit einem Gewindeabschnitt 12 und einem Schlüsselsechskant 13 versehen ist. In dem Gehäuse 11 ist ein Sensorelement 14 so verbaut, dass ein Endabschnitt 141 aus dem Gehäuse 11 herausragt. Die Verbauung im Gehäuse 11 erfolgt mittels eines Dichtelements 15, das im Ausführungsbeispiel von einer Dichtungspackung, bestehend aus einem zwischen zwei Keramikformteilen axial verpressten elastischen Dichtung 16, die sich radial an das Sensorelement 14 und an die Innenwand des Gehäuses 11 anpresst, gebildet ist. In Fig. 1 ist lediglich das am messgasseitigen Ende des Gehäuses 11 angeordnete Keramikformteil 17 dargestellt, das eine zentrale rechteckförmige Öffnung 171 für den Durchtritt des Sensorelements 14 aufweist und sich an einer im Gehäuse 11 ausgebildeten Radialschulter 111 axial abstützt. Auf dem gassensitiven Endabschnitt 141 des z.B. einen stabförmigen Keramikkörper aufweisenden Sensorelements 14 ist auf einer Großfläche des Keramikkörpers eine sog. Interdigitalelektrode 18 zur Messung einer auf dem Endabschnitt 141 herbeigeführten Rußablagerung angeordnet. Die Interdigitalelektrode 18 weist zwei kammartig ausgebildete und mit den Kammzähnen ineinandergreifende Elektrodenabschnitte auf. Die Wirkungsweise und der Aufbau einer solchen Interdigitalelektrode zur Bestimmung der sich auf ihr ablagernden Rußmenge als Maß für die Rußkonzentration im Abgas ist in der DE 10 2004 028 997 A1 beschrieben.

Der gassensitive Endabschnitt 141 des Sensorelements 14 ist von einem Schutzrohrmodul 20 abgedeckt, wobei das Schutzrohrmodul 20 mit Mitteln für den Gasdurchtritt versehen ist, sodass das in dem Messgas-Strömungskanal bzw. in dem Abgasrohr der Brennkraftmaschine strömende Mess- bzw. Abgas an den gassensitiven Endabschnitt 141 gelangen kann. Die Strömungsrichtung des Mess- oder Abgases ist in Fig. 1 mit den Strömungspfeilen 19 symbolisiert. Das Schutzrohrmodul 20 ist zusammengesetzt aus einem den Endabschnitt 141 des Sensorelements 14 mit Radial- und Axialabstand umschließenden, hutförmigen Innenschutzrohr 21 und aus einem das Innenschutzrohr mit Radialabstand umgebenden, kappen- oder topfförmigen Außenschutzrohr 22. Das hutförmige Innenschutzrohr 21 weist einen Hutboden 211, eine Hutöffnung 212 und eine die Hutöffnung 212 umgebende Hutkrempe 213 auf. Der Hutboden 211 und die Hutkrempe 213 sind über den zylindermantelförmigen Hutmantel 214 verbunden. Der äußere Rand 213a der Hutkrempe 213 ist rechtwinklig abgebogen und übergreift einen an der Stirnseite des Gehäuses 11 einstückig angeformten Befestigungsstutzen 112, dessen Außendurchmesser gegenüber dem Außendurchmesser des Gehäuses 11 reduziert ist. Das topfförmige Außenschutzrohr 22 weist einen Topfboden 221 mit einer zentralen kreisförmigen Aussparung 23 und einen Topfmantel 222 auf, der über den abgewinkelten Rand 213a der Hutkrempe 213 des Innenschutzrohrs 21 geschoben ist, sodass zwischen dem Innenschutzrohr 21 und dem Außenschutzrohr 22 ein Ringraum 33 vorhanden ist, dessen radiale Breite der Breite der Hutkrempe 213 entspricht. Die axiale Länge des Außenschutzrohrs 22 ist deutlich kleiner als die axiale Länge des Innenschutzrohrs 21, sodass letzteres durch die kreisförmige Aussparung 23 im Topfboden hindurchtritt und deutlich über den Topfboden 221 vorsteht. Das Schutzrohrmodul 20 ist auf dem Befestigungsstutzen 112 des Gehäuses 11 stoffschlüssig festgelegt, z.B. durch eine umlaufende Schweißnaht.

Die im Schutzrohrmodul 20 vorgesehenen Mittel für den Gasdurchtritt umfassen einen Gasaustritt 24 im Innenschutzrohr 21, der beispielhaft von einem zentralen Loch 25 im Hutboden 211 des Innenschutzrohrs 21 gebildet ist, einen Gaseintritt 26 im Außenschutzrohr 22 und einen Gaseintritt 27 im Innenschutzrohr 21. Der Gaseintritt 26 im Außenschutzrohr 22 ist durch ein eine oder mehrere im Topfmantel 222 ausgebildete Öffnungen realisiert, insbesondere als ein eine oder mehrere Löcher und/oder als ein eine oder mehrere Drallklappen. Der Gaseintritt 27 im Innenschutzrohr 21 ist in die Hutkrempe 213 gelegt, und in Strömungsrichtung des in das Schutzrohrmodul 20 einströmenden Messgases hinter der Hutkrempe 213, also zum Gehäuse 11 hin, ist ein bis unter die Hutöffnung 212 reichender Freiraum 29 vorgesehen. Der Gaseintritt 27 ist mit Durchbrüchen 30 in der Hutkrempe 213 realisiert, die in Umfangsrichtung der Hutkrempe 213 voneinander beabstandet angeordnet sind. Vorzugsweise sind die Durchbrüche 30 als kreisförmige Löcher ausgebildet. Im Freiraum 29 sind Strömungsmittel angeordnet, die die durch die Durchbrüche 30 hindurchtretende Messgasströmung zur Hutöffnung 212 hin umlenken.

Durch die im Topfmantel ausgebildeten Öffnungen beziehungsweise Drallklappen kommt die Wirkung zustande, dass Abgas auch bei langsam strömendem Abgas mit hoher Dynamik in das Innere des Sensors und an das Sensorelement 14 gelangen kann, wenngleich ein Schutz des Sensorelements 14 vor dem Auftreffen von flüssigen Bestandteilen des Abgases, beispielsweise Wassertropfen, weiterhin gegeben ist.

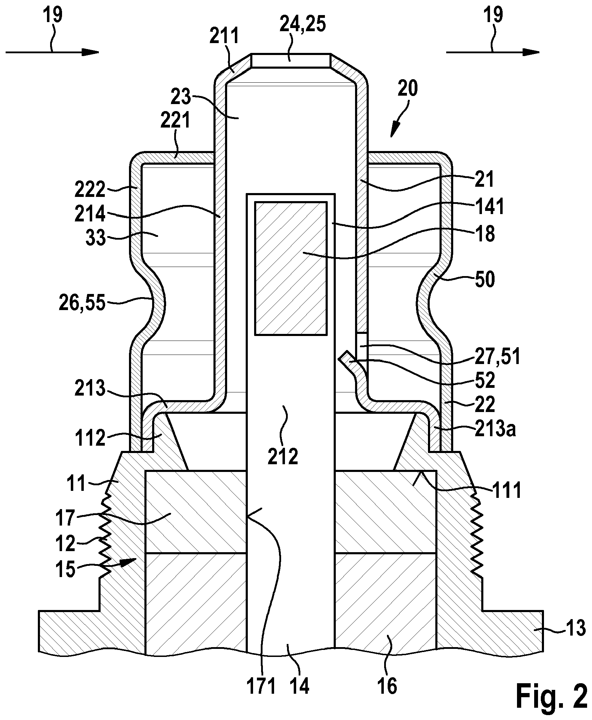

In der Figur 2 ist ein dem Abgas zugewandter Abschnitt eines Gassensors gemäß der zweiten Konzeption, die nicht unter den Schutzumfang des Anspruchs fällt, dargestellt. Es handelt sich um einen Sensor zur Bestimmung der Partikel, insbesondere Rußkonzentration im Abgas einer Brennkraftmaschine, auch Partikel- oder Rußsensor genannt Er wird als Beispiel für einen allgemeinen Gassensor zur Bestimmung mindestens einer Zustandsgröße eines Messgases angegeben. Andere Gassensoren dieser Art sind Gassensoren zur Bestimmung der Sauerstoffkonzentration im Abgas einer Brennkraftmaschine, sog. Lambdasonden, oder Gassensoren zur Bestimmung der Stickoxidkonzentration im Abgas einer Brennkraftmaschine. Auch Temperaturmessfühler zur Messung der Abgastemperatur können ein solcher Gassensor sein.

Der der Figur 2 dargestellte Gassensor weist ein metallisches Gehäuse 11 auf, das zum Einbau in einen hier nicht dargestellten Strömungskanal für das Messgas, insbesondere in das Abgasrohr eines Brenners oder einer Brennkraftmaschine, mit einem Gewindeabschnitt 12 und einem Schlüsselsechskant 13 versehen ist. In dem Gehäuse 11 ist ein Sensorelement 14 so verbaut, dass ein Endabschnitt 141 aus dem Gehäuse 11 herausragt. Die Verbauung im Gehäuse 11 erfolgt mittels eines Dichtelements 15, das im Ausführungsbeispiel von einer Dichtungspackung, bestehend aus einem zwischen zwei Keramikformteilen axial verpressten elastischen Dichtung 16, die sich radial an das Sensorelement 14 und an die Innenwand des Gehäuses 11 anpresst, gebildet ist. In Fig. 2 ist lediglich das am messgasseitigen Ende des Gehäuses 11 angeordnete Keramikformteil 17 dargestellt, das eine zentrale rechteckförmige Öffnung 171 für den Durchtritt des Sensorelements 14 aufweist und sich an einer im Gehäuse 11 ausgebildeten Radialschulter 111 axial abstützt. Auf dem gassensitiven Endabschnitt 141 des z.B. einen stabförmigen Keramikkörper aufweisenden Sensorelements 14 ist auf einer Großfläche des Keramikkörpers eine sog. Interdigitalelektrode 18 zur Messung einer auf dem Endabschnitt 141 herbeigeführten Rußablagerung angeordnet. Die Interdigitalelektrode 18 weist zwei kammartig ausgebildete und mit den Kammzähnen ineinandergreifende Elektrodenabschnitte auf. Die Wirkungsweise und der Aufbau einer solchen Interdigitalelektrode zur Bestimmung der sich auf ihr ablagernden Rußmenge als Maß für die Rußkonzentration im Abgas ist in der DE 10 2004 028 997 A1 beschrieben.

Der gassensitive Endabschnitt 141 des Sensorelements 14 ist von einem Schutzrohrmodul 20 abgedeckt, wobei das Schutzrohrmodul 20 mit Mitteln für den Gasdurchtritt versehen ist, sodass das in dem Messgas-Strömungskanal bzw. in dem Abgasrohr der Brennkraftmaschine strömende Mess- bzw. Abgas an den gassensitiven Endabschnitt 141 gelangen kann. Die Strömungsrichtung des Mess- oder Abgases ist in Fig. 1 mit den Strömungspfeilen 19 symbolisiert. Das Schutzrohrmodul 20 ist zusammengesetzt aus einem den Endabschnitt 141 des Sensorelements 14 mit Radial- und Axialabstand umschließenden, hutförmigen Innenschutzrohr 21 und aus einem das Innenschutzrohr mit Radialabstand umgebenden, kappen- oder topfförmigen Außenschutzrohr 22. Das hutförmige Innenschutzrohr 21 weist einen Hutboden 211, eine Hutöffnung 212 und eine die Hutöffnung 212 umgebende Hutkrempe 213 auf. Der Hutboden 211 und die Hutkrempe 213 sind über den zylindermantelförmigen Hutmantel 214 verbunden. Der äußere Rand 213a der Hutkrempe 213 ist rechtwinklig abgebogen und übergreift einen an der Stirnseite des Gehäuses 11 einstückig angeformten Befestigungsstutzen 112, dessen Außendurchmesser gegenüber dem Außendurchmesser des Gehäuses 11 reduziert ist. Das topfförmige Außenschutzrohr 22 weist einen Topfboden 221 mit einer zentralen kreisförmigen Aussparung 23 und einen Topfmantel 222 auf, der über den abgewinkelten Rand 213a der Hutkrempe 213 des Innenschutzrohrs 21 geschoben ist, sodass zwischen dem Innenschutzrohr 21 und dem Außenschutzrohr 22 ein Ringraum 33 vorhanden ist, dessen radiale Breite der Breite der Hutkrempe 213 entspricht. Die axiale Länge des Außenschutzrohrs 22 ist deutlich kleiner als die axiale Länge des Innenschutzrohrs 21, sodass letzteres durch die kreisförmige Aussparung 23 im Topfboden hindurchtritt und deutlich über den Topfboden 221 vorsteht. Das Schutzrohrmodul 20 ist auf dem Befestigungsstutzen 112 des Gehäuses 11 stoffschlüssig festgelegt, z.B. durch eine umlaufende Schweißnaht.

Die im Schutzrohrmodul 20 vorgesehenen Mittel für den Gasdurchtritt umfassen einen Gasaustritt 24 im Innenschutzrohr 21, der beispielhaft von einem zentralen Loch 25 im Hutboden 211 des Innenschutzrohrs 21 gebildet ist, einen Gaseintritt 26 im Außenschutzrohr 22 und einen Gaseintritt 27 im Innenschutzrohr 21. Der Gaseintritt 26 im Außenschutzrohr 22 ist durch ein, zwei oder mehr als zwei im Topfmantel 222 ausgebildete Öffnungen realisiert, insbesondere als ein, zwei oder mehr als zwei Löcher und/oder als ein, zwei oder mehr als zwei Drallklappen 55. Insbesondere können zwei Drallklappen 55 vorgesehen sein, die eine Strömung in zueinander tangential entgegengesetzte Richtungen umleiten. Der Gaseintritt 27 im Innenschutzrohr 21 ist in den Hutmantel 214 gelegt, insbesondere in die dem abgasseitigen Ende des Gassensors abgewandte Hälfte des Hutmantels, in Figur 2 unten. Der Gaseintritt 27 im Innenschutzrohr 21 ist als eine einzige Öffnung 51 des Hutmantels 214 realisiert. Das Außenschutzrohr 22 kann im Bereich dieser einzigen Öffnung 51 des Hutmantels 214 eine Einbuchtung 50 aufweisen, sodass das Volumen bzw. die Breite des sich zwischen den Schutzrohren 21, 22 befindlichen Ringraums 33 an dieser Stelle reduziert ist.

Vorteilhafterweise ist am Rande des Gaseintritts 27, im Hutmantel 214 ein nach innen geneigtes, strömungsführendes Element 52 vorgesehen, dass die Gasströmung zusätzlich in das Innere des inneren Schutzrohrs 21 umlenkt.

Es ist insbesondere vorgesehen, dass sich der Gaseintritt 27, vorliegend die einzige Öffnung 51 des Hutmantels 214, und der Gaseintritt 26 im Außenschutzrohr 22 auf einander radial gegenüberliegenden Seiten des Schutzrohrmoduls 20 ausgebildet sind, wobei auf der Seite des Gaseintritts 27, vorliegend die einzige Öffnung 51 des Hutmantels 214, kein Gaseintritt 26 im Außenschutzrohr 22 vorgesehen ist.

Es ist insbesondere vorgesehen, dass sich der Gaseintritt 27, vorliegend die einzige Öffnung 51 des Hutmantels 214, und die auf der Oberfläche des Sensorelements 14 angeordnete Interdigitalelektrode 18 so angeordnet sind, dass sie in die gleiche radiale Richtung weisen.

Vorzugsweise umfasst der Sensor Mittel zu seinem gerichteten Verbau, wie etwa Markierungen, Rastmittel, Überwurfmuttern, Bajonettverschlüsse und/oder ähnliches, sodass der Gaseintritt 27, vorliegend die einzige Öffnung 51 des Hutmantels 214, auf einer stromabwärts gelegenen Seite in einem Abgastrakt verbaut werde kann.

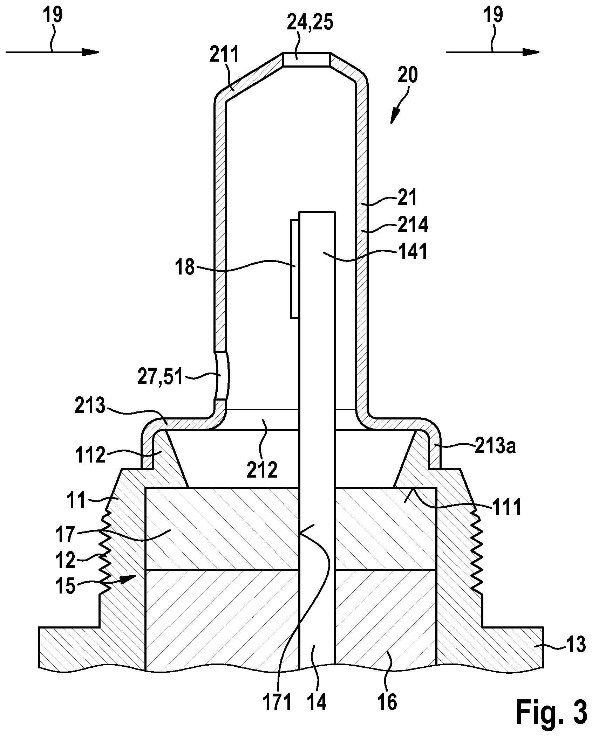

In der Figur 3 ist ein dem Abgas zugewandter Abschnitt eines Gassensors gemäß der dritten Konzeption, die nicht unter den Schutzumfang des Anspruchs fällt, dargestellt. Es handelt sich um einen Sensor zur Bestimmung der Partikel, insbesondere Rußkonzentration im Abgas einer Brennkraftmaschine, auch Partikel- oder Rußsensor genannt. Er wird als Beispiel für einen allgemeinen Gassensor zur Bestimmung mindestens einer Zustandsgröße eines Messgases angegeben. Andere Gassensoren dieser Art sind Gassensoren zur Bestimmung der Sauerstoffkonzentration im Abgas einer Brennkraftmaschine, sog. Lambdasonden, oder Gassensoren zur Bestimmung der Stickoxidkonzentration im Abgas einer Brennkraftmaschine. Auch Temperaturmessfühler zur Messung der Abgastemperatur können ein solcher Gassensor sein.

Der der Figur 3 dargestellte Gassensor weist ein metallisches Gehäuse 11 auf, das zum Einbau in einen hier nicht dargestellten Strömungskanal für das Messgas, insbesondere in das Abgasrohr eines Brenners oder einer Brennkraftmaschine, mit einem Gewindeabschnitt 12 und einem Schlüsselsechskant 13 versehen ist. In dem Gehäuse 11 ist ein Sensorelement 14 so verbaut, dass ein Endabschnitt 141 aus dem Gehäuse 11 herausragt. Die Verbauung im Gehäuse 11 erfolgt mittels eines Dichtelements 15, das im Ausführungsbeispiel von einer Dichtungspackung, bestehend aus einem zwischen zwei Keramikformteilen axial verpressten elastischen Dichtung 16, die sich radial an das Sensorelement 14 und an die Innenwand des Gehäuses 11 anpresst, gebildet ist. In Fig. 3 ist lediglich das am messgasseitigen Ende des Gehäuses 11 angeordnete Keramikformteil 17 dargestellt, das eine zentrale rechteckförmige Öffnung 171 für den Durchtritt des Sensorelements 14 aufweist und sich an einer im Gehäuse 11 ausgebildeten Radialschulter 111 axial abstützt. Auf dem gassensitiven Endabschnitt 141 des z.B. einen stabförmigen Keramikkörper aufweisenden Sensorelements 14 ist auf einer Großfläche des Keramikkörpers eine sog. Interdigitalelektrode 18 zur Messung einer auf dem Endabschnitt 141 herbeigeführten Rußablagerung angeordnet. Die Interdigitalelektrode 18 weist zwei kammartig ausgebildete und mit den Kammzähnen ineinandergreifende Elektrodenabschnitte auf. Die Wirkungsweise und der Aufbau einer solchen Interdigitalelektrode zur Bestimmung der sich auf ihr ablagernden Rußmenge als Maß für die Rußkonzentration im Abgas ist in der DE 10 2004 028 997 A1 beschrieben.

Der gassensitive Endabschnitt 141 des Sensorelements 14 ist von einem Schutzrohrmodul 20 abgedeckt, wobei das Schutzrohrmodul 20 mit Mitteln für den Gasdurchtritt versehen ist, sodass das in dem Messgas-Strömungskanal bzw. in dem Abgasrohr der Brennkraftmaschine strömende Mess- bzw. Abgas an den gassensitiven Endabschnitt 141 gelangen kann. Die Strömungsrichtung des Mess- oder Abgases ist in Fig. 1 mit den Strömungspfeilen 19 symbolisiert. Das Schutzrohrmodul 20 besteht aus einem den Endabschnitt 141 des Sensorelements 14 mit Radial- und Axialabstand umschließenden, hutförmigen Innenschutzrohr 21. Ein weiteres, das Innenschutzrohr umgebendes Außenschutzrohr ist nicht vorgesehen. Das hutförmige Innenschutzrohr 21 weist einen Hutboden 211, eine Hutöffnung 212 und eine die Hutöffnung 212 umgebende Hutkrempe 213 auf. Der Hutboden 211 und die Hutkrempe 213 sind über einen zylindermantelförmigen Hutmantel 214 verbunden. Der äußere Rand 213a der Hutkrempe 213 ist rechtwinklig abgebogen und übergreift einen an der Stirnseite des Gehäuses 11 einstückig angeformten Befestigungsstutzen 112, dessen Außendurchmesser gegenüber dem Außendurchmesser des Gehäuses 11 reduziert ist.

Die im Schutzrohrmodul 20 vorgesehenen Mittel für den Gasdurchtritt umfassen einen Gasaustritt 24 im Innenschutzrohr 21, der beispielhaft von einem Loch 25 im Hutboden 211 des Innenschutzrohrs 21 gebildet ist. Der Gaseintritt 27 im Innenschutzrohr 21 ist in den Hutmantel 214 gelegt und als eine einzige Öffnung 51 des Hutmantels 214 realisiert. Sie befindet sich in der der Hutkrempe 213 zugewandten Hälfte des Hutmantel 214, vorzugsweise im der Hutkrempe 213 zugewandten Drittel oder Viertel des Hutmantels 214, in Figur 3 unten.

Es ist insbesondere vorgesehen, dass sich der Gaseintritt 27, vorliegend die einzige Öffnung 51 des Hutmantels 214, auf einer radialen Seite des Schutzrohrmoduls 20 ausgebildet ist, während das Sensorelement 14 zur gegenüberliegen Seite hin radial außermittig im Gehäuse 11 und/oder in dem Schutzrohrmoduls 20 angeordnet ist.

Es ist insbesondere vorgesehen, dass sich der Gaseintritt 27, vorliegend die einzige Öffnung 51 des Hutmantels 214, auf einer radialen Seite des Schutzrohrmoduls 20 ausgebildet ist, während die Gasaustrittsöffnung 24 als Loch 25 im Hutboden 211 zur gegenüberliegen Seite hin radial außermittig ausgebildet ist. Insbesondere liegen das radial außermittig angeordnete Sensorelement 14 und die radial außermittig angeordnete Gasaustrittsöffnung 24 in radialer Aufsicht übereinander.

Es ist insbesondere vorgesehen, dass sich der Gaseintritt 27, vorliegend die einzige Öffnung 51 des Hutmantels 214, und die auf der Oberfläche des Sensorelements 14 angeordnete Interdigitalelektrode 18 so angeordnet sind, dass sie in die gleiche radiale Richtung weisen.

Vorzugsweise umfasst der Sensor Mittel zu seinem gerichteten Verbau, wie etwa Markierungen, Rastmittel, Überwurfmuttern, Bajonettverschlüsse und/oder ähnliches, sodass der Gaseintritt 27, vorliegend die einzige Öffnung 51 des Hutmantels 214, auf der stromabwärts gelegenen Seite verbaut werde kann.

## Patentansprüche

1. Gassensor zur Bestimmung mindestens einer Zustandsgröße eines Messgases, insbesondere der Partikelkonzentration im Abgas einer Brennkraftmaschine oder eines Brenners, mit einem in einem Gehäuse (11) verbauten Sensorelement (14), das einen aus dem Gehäuse (11) vorstehenden, einem Messgasstrom ausgesetzten, gassensitiven Endabschnitt (141) aufweist, mit einem den Endabschnitt (141) abdeckenden, am Gehäuse (11) festgelegten Schutzrohrmodul (20), das ein den Endabschnitt (141) mit Radial- und Axialabstand umschließendes, hutförmiges Innenschutzrohr (21) mit Hutboden (211), Hutöffnnung (212) und Hutkrempe (213) und ein das Innenschutzrohr (21) mit Radialabstand umgebendes, topfförmiges Außenschutzrohr (22) mit gegenüber dem Hutboden (111) zurückversetztem Topfboden (221) ohne Gaseintritt (26) und einem Topfmantel (222) aufweist, und mit im Schutzrohrmodul (20) vorgesehenen Mitteln für den Gasdurchtritt, die einen im Hutboden (211) des Innenschutzrohrs (21) vorhandenen Gasaustritt (24) und im Außenschutzrohr (22) und im Innenschutzrohr (21) vorhandene Gaseintritte (26, 27) aufweisen, wobei der im Außenschutzrohr (22) vorhandene Gaseintritt (26) im Topfmantel (222) angeordnet und als Drallklappe ausgebildet ist, **dadurch gekennzeichnet, dass** der Gaseintritt (27) im Innenschutzrohr (21) in die Hutkrempe (213) gelegt und in Strömungsrichtung des Messgases hinter der Hutkrempe (213) ein bis unter die Hutöffnung (212) reichender Freiraum (29) zur Umlenkung der Messgasströmung in das Innenschutzrohr (21) vorhanden ist.

## Claims

1. Gas sensor for determining at least one state variable of a measurement gas, in particular the particle concentration in the exhaust gas of an internal combustion engine or a burner, comprising a sensor element (14) which is installed in a housing (11) and which has a gas-sensitive end portion (141) projecting from the housing (11) and exposed to a measurement gas flow, comprising a protective tube module (20) which covers the end portion (141) and which is fixed on the housing (11), said module having a hat-shaped inner protective tube (21) which surrounds the end portion (141) with a radial and axial spacing and which has a hat base (211), hat opening (212) and hat brim (213) and having a pot-shaped outer protective tube (22) which surrounds the inner protective tube (21) with a radial spacing and which has a pot base (221) without a gas inlet (26), said pot base being set back in relation to the hat base (111), and which has a pot casing (222), and comprising means for the gas passage which are provided in the protective tube module (20) and which have a gas outlet (24) which is present in the hat base (211) of the inner protective tube (21) and gas inlets (26, 27) which are present in the outer protective tube (22) and in the inner protective tube (21), wherein the gas inlet (26) which is present in the outer protective tube (22) is arranged in the pot casing (222) and is in the form of a swirl flap, **characterized in that** the gas inlet (27) in the inner protective tube (21) is placed into the hat brim (213), and a free space (29) which extends to below the hat opening (212) and which serves for deflection of the measurement gas flow into the inner protective tube (21) is present behind the hat brim (213) in the flow direction of the measurement gas.

## Revendications

1. Capteur de gaz servant à déterminer au moins une grandeur d'état d'un gaz à mesurer, en particulier de la concentration en particules dans les gaz d'échappement d'un moteur à combustion interne ou d'un brûleur, le capteur présentant
un élément de capteur (14) monté dans un boîtier (11) et dont une section d'extrémité (141) sensible au gaz déborde du boîtier (11) et est exposée à un écoulement de gaz à mesurer,
un module tubulaire de protection (20) recouvrant la section d'extrémité (141) et fixé sur le boîtier (11), présentant un tube intérieur de protection (21) en forme de chapeau entourant la section d'extrémité (141) à distance radiale et à distance axiale présentant un fond de chapeau (211), une ouverture de chapeau (212) et un rebord de chapeau (213), et
un tube extérieur de protection (22) en forme de cuvette, entourant le tube intérieur de protection (21) à une distance radiale et présentant un fond de cuvette (221) sans entrée de gaz (26) et en retrait par rapport au fond de chapeau (111), une enveloppe de cuvette (222), et des moyens prévus dans le module de protection (20) pour le passage du gaz, qui présente une sortie de gaz (24) prévue dans le fond de chapeau (211) du tube intérieur de protection (21) et des entrées de gaz (26, 27) prévues dans le tube extérieur de protection (22) et le tube intérieur de protection (21), l'entrée de gaz (26) prévue dans le tube extérieur de protection (22) étant disposée dans l'enveloppe de cuvette (222) et configurée comme clapet de tourbillonnage,
**caractérisé en ce que**
l'entrée de gaz (27) prévue dans le tube intérieur de protection (21) est placée dans le rebord de chapeau (213) et un espace libre (29) qui s'étend jusqu'en dessous de l'ouverture de chapeau (212) est prévu en aval du rebord de chapeau (213) dans la direction d'écoulement du gaz à mesurer pour dévier dans le tube intérieur de protection (21) l'écoulement de gaz à mesurer.
